# EUROPEAN PATENT APPLICATION

(11) **EP 1 806 716 A1**
(43) Date of publication of application: **11.07.2007**
(21) Application number: 06123289.8
(22) Date of filing: 31.10.2006
(51) Int. Cl.: G09G 3/28

(54) **Plasma display panel apparatus**

(30) Priority: 06.01.2006 KR 20060002012
(71) Applicant: SAMSUNG ELECTRONICS CO., LTD., Suwon-si, Gyeonggi-Do 442-742 (KR)
(72) Inventor: Kim, Jong-hyun 942-1204, Byeokjeokgol 9danji, Suwon-si Gyeonggi-do (KR); Ahn, Jung-hyun 204-1504, Unam Firstvill, Gyeonggi-do (KR)
(74) Representative: Walaski, Jan Filip

(57) **Abstract**

A plasma display panel (PDP) apparatus and a method of updating the PDP apparatus are provided. The PDP apparatus includes: a PDP module which displays an image; and a signal processing module which processes audio and/or video signals provided from a signal source and provides the connected PDP module with an update program from an external apparatus. Accordingly, it is possible to update the program of the PDP module without separating the PDP module from the signal processing module.

## Description

The present invention relates to a plasma display panel (PDP) apparatus and more particularly, but not exclusively, to a PDP apparatus capable of being updated, and a method of updating a PDP module of a PDP apparatus without separating it from a signal processing module of the PDP apparatus.

A PDP is a next-generation type display with large-size, high-contrast, high-clarity and super-lightness, and is employed as various apparatuses such as a television and a monitor.

The PDP apparatus is generally divided into a signal processing module and a PDP module. The signal processing module produces a viewable signal from at least one of audio signals and video signals received by a signal receiving terminal such as a tuner. The PDP module controls on and off operations of numerous cells formed between two glass panels, to display a real image. Each module is driven by using an appropriate application program.

However, after the signal processing module and the PDP module are manufactured, there may be a need to update each application program. The signal processing module of a related art PDP apparatus is connected to a computer, in order to update the application program. However, the PDP module needs to be separated from the signal processing module and requires a separate download tool, to update the program. That is, inconveniently, a developer separates the PDP module and connects the separate download tool to the separated PDP module, in order to update the PDP module.

In particular, when the PDP module is mass-produced by a manufacturer, the developer either needs be supplied with the download tool from the manufacturer or needs to send the separated PDP module to the manufacturer, in order to update the application program of the PDP module.

An embodiment of the present invention provides a PDP apparatus capable of updating and an updating method thereof, in which it is possible to update a PDP module without separating the PDP module, and in which the PDP module is connected to a signal processing module when a product is being developed or completed.

According to an aspect of the present invention, there is provided a PDP apparatus capable of updating comprising: a PDP module implementing an image; and a signal processing module processing at least one of audio signals and video signals provided from a signal source and providing the connected PDP module with an update program of the PDP module provided from an external apparatus.

The signal processing module may comprise: a user selecting unit outputting a first mode selecting signal for updating the PDP module; a first interfacing unit communicating with the PDP module; a second interfacing unit communicating with the external apparatus; a first controlling unit outputting the first controlling signal to provide a communication path with the PDP module, if the first mode selecting signal is received; and a switching unit whose first end is connected to the first interfacing unit and whose second end switches the second interfacing unit based on the output first controlling signal and provides a communication path between the external apparatus and the PDP module.

The first controlling unit outputs to the switching unit the second controlling signal to switch off the communication path between the external apparatus and the PDP module if the second mode selecting signal for updating the signal processing module is received from the user selecting unit, and directly receives the update program provided from the external apparatus through the second interfacing unit.

The PDP module may comprise: a third interfacing unit receiving the update program provided from the external apparatus through the signal processing module; a storing unit storing the received update program; and a second controlling unit controlling the third interfacing unit and the storing unit to receive and store the update program.

The signal processing module may be connected to the external apparatus through one of a computer cable, an RS (recommended standard)-232 cable, and a universal serial bus.

When the signal processing module is connected to the external apparatus a the computer cable, the external apparatus comprises: an external computer providing the update program of the PDP module; and a DDC (display data channel) manager performing a relay role between the second interfacing unit and the external computer and transmitting the update program of the PDP module to the signal processing module.

When the signal processing module is connected to the external apparatus through one of an RS-2332 cable and a universal serial bus, the external apparatus is an external computer stored with the update program of the PDP module.

According to another aspect of the present invention, there is provided an updating method of a PDP apparatus capable of updating which comprises a PDP module implementing an image and a signal processing module processing at least one of audio signals and video signals, the updating method comprising: selecting a mode for updating the PDP module; providing the PDP module with the update program of the PDP module provided from an external apparatus connected to the signal processing module, if the mode is selected: and storing the update program in the PDP module.

The providing the PDP module with the update program may comprise: outputting a control signal to provide a communication path between the PDP module and the external apparatus, if the mode is selected; switching the external apparatus according to the control signal and providing the communication path; and loading the PDP module with the update program of the PDP module provided from the external apparatus through the communication path.

The signal processing module may be connected to the external apparatus through one of a computer cable, RS (recommended standard)-232 cable and a universal serial bus, and connected to the PDP module through a cable applied with an Inter-IC communication standard.

According to another aspect of the present invention, there is provided a PDP displaying apparatus comprising: a PDP module implementing an image; and a signal processing module processing at least one of audio signals and video signals provided from a signal source and storing an update program of the PDP module provided from an external apparatus.

The signal processing module may comprise: a user selecting unit outputting a mode selecting signal for updating the PDP module; a first interfacing unit communicating with the PDP module; a second interfacing unit communicating with the external apparatus; a storing unit storing the update program of the PDP module; and a controlling unit the second interfacing unit and the storing unit to receive and store the update program provided from the external apparatus, if the mode selecting signal is received.

The controlling unit controls the storing unit and the first interfacing unit to provide the PDP module with the update program stored in the storing unit, to update the PDP module, if there is a request for updating the PDP module from the user selecting unit.

The signal processing module may be connected to the external apparatus through one of a computer cable, RS (recommended standard)-232 cable and a universal serial bus.

According to another aspect of the present invention, there is provided an updating method of a PDP displaying apparatus comprising a PDP module implementing an image, and a signal processing module processing at least one of audio signals and video signals, the updating method comprises: selecting a mode for updating the PDP module; receiving an update program of the PDP module provided from an external apparatus connected to the signal processing module; and storing the received update program in the signal processing module.

The updating method may further comprise requesting updating the PDP module; and updating the PDP module by transmitting the stored update program to the PDP module.

Embodiments of the present invention will now be described, by way of example only, with reference to the accompanying drawings, in which:
Figure 1 is a block diagram of a PDP apparatus according to a first embodiment of the present invention;
Figure 2 is a block diagram of a PDP apparatus according to a second embodiment of the present invention;
Figure 3 is a flowchart of an updating method of the PDP apparatus according to the first embodiment of the present invention;
Figure 4 is a block diagram of a PDP apparatus according to a third embodiment of the present invention;
Figure 5 is a block diagram of a PDP apparatus according to a fourth embodiment of the present invention; and
Figure 6 is a flowchart of an updating method of the PDP apparatus according to the third embodiment of the present invention.

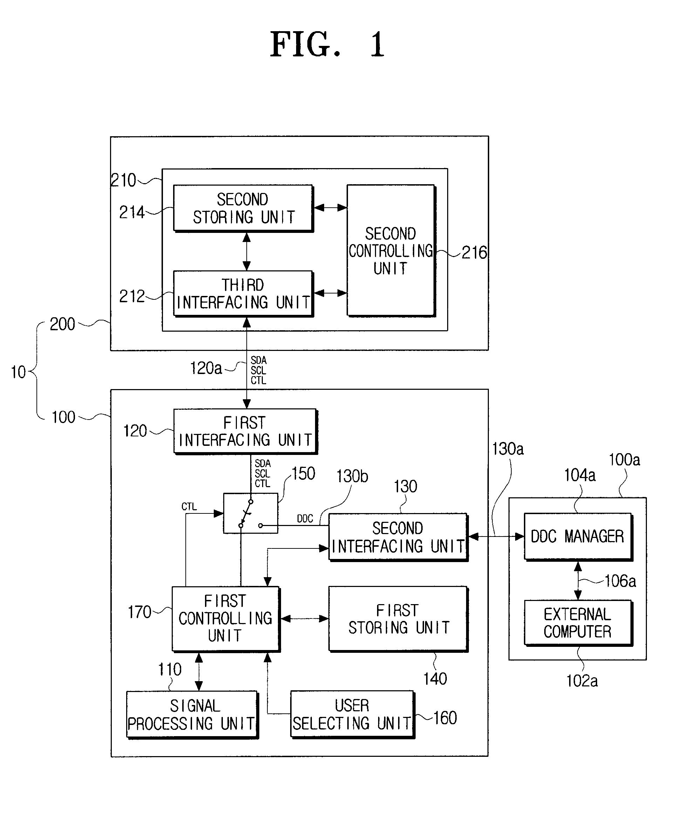

Figure 1 is a block diagram of a PDP apparatus according to the first embodiment of the present invention.

Referring to Figure 1, the PDP apparatus 10 capable of being updated according to the first embodiment of the present invention, includes a signal processing module 100 and a PDP module 200, and updates a software program of the signal processing module 100 and the PDP module 200 using update programs provided by an external apparatus 100a. The update programs are transmitted from the external apparatus 100a to the PDP module 200, based on Inter-IC (hereinafter, referred to as "I2C") communication standards.

The external apparatus 100a includes an external computer 102a and a display data channel (DDC) manager 104a. The external computer 102a stores the update programs of the signal processing module 100 and the PDP module 200. The DDC manager 104a performs a role of relay between the external computer 102a and the signal processing module 100.

The DCC manager 104a is connected to the external computer 102a through a line print terminal (LPT) port cable 106a, and connected to the second interface unit 130 of the signal processing module 100 through a computer cable 130a. However, various changes in form and details may be made therein without departing from the scope of the invention as defined by the appended claims.

The signal processing module 100 is connected to the external apparatus 100a to receive the update programs of the signal processing module 100 and the PDP module 200 according to a mode of operation of the PDP apparatus 10, and provides a path for transmitting the update program of the PDP module 200 to the PDP module 200.

Towards this goal, the signal processing module 100 includes a signal processing unit 110, the first interfacing unit 120, the second interfacing unit 130, the first storing unit 140, a switching unit 150, a user selecting unit 160, and the first controlling unit 170.

The signal processing unit 110 processes at least one of audio signals and video signals provided from a signal source into a viewable signal. The signal source may be, for example, a tuner, a digital video disc (DVD) player or a satellite broadcast receiver.

The first interfacing unit 120 provides a communication path with the PDP module 200, and may be a low voltage differential signalling (LVPS) jack, according to this embodiment. The first interfacing unit 120 and the PDP module 200 are communicably connected through the LVPS cable 120a using I2C communication standards.

The second interfacing unit 130 provides the communication path with the external apparatus 100a, and may be a computer jack or a service port according to this embodiment. The second interfacing unit 130 is communicably connected to the DDC manager 104a through the computer cable 130a, and connected to the switching unit 150 through a DDC line 130b compliant with the I2C communication standards.

The first storing unit 140 stores the application program of the signal processing module 100, and updates an existing program based on the update program provided from the external apparatus 100a. The first storing unit 140 may be, for example, a flash memory or a synchronous dynamic random access memory (SDRAM).

The user selecting unit 160 includes a plurality of number keys, direction keys and confirmation keys for selecting functions supported by the PDP apparatus 10, and provides the first controlling unit 170 with a signal corresponding to a key or keys selected by a user.

The switching unit 150 connects the second interfacing unit 130 or the first controlling unit 170 to the first interfacing unit 120 based on a control signal of the first controlling unit 170. More particularly, the switching unit 150 connects the first interfacing unit 120 to the second interfacing unit 130 to provide the communication path between the PDP module 200 and the external apparatus 100a, or connects the first interfacing unit 120 to the first controlling unit 170 to provide the communication path between the PDP module 200 and the first controlling unit 170.

According to this embodiment, the user selecting unit 160 may select a first mode or a second mode of operation of the PDP apparatus 10. In the first mode, updating of the PDP module 200 is requested, and in the second mode, updating of the signal processing module 100 is requested.

When the first mode selection signal is received from the user selecting unit 160, the first controlling unit 170 outputs a first control signal (CTL) to the switching unit 150 and the first interfacing unit 120 to provide the communication path between the PDP module 200 and the external apparatus 100a. The update program of the PDP module 200 is transmitted to the PDP module 200 through the second interfacing unit 130, the switching unit 150, the first interfacing unit 120 and the LVPS cable 120a.

The external computer 102a generates the update program of the PDP module 200 which includes data (SDA) and a clock signal (SCL), in accordance with the 12C standards, and transmits the generated update program to the signal processing module 100. The update program, including the data SDA and the clock signal SCL and the first control signal CTL, is transmitted to the PDP module 200 by the signal processing module 100.

When the second mode selection signal is received from the user selecting unit 160, the first controlling unit 170 outputs a second control signal (CTL) to the switching unit 150 and the first interfacing unit 120, to disconnect the communication path between the first interfacing unit 120 and the second interfacing unit 130 and connect the first controlling unit 170 to the PDP module 200 through the first interfacing unit 120. The update program of the signal processing module 100 provided by the external computer 102a, is provided to the first storage unit 140 through the second interfacing unit 130 and the first controlling unit 170.

The PDP module 200 turns on and off cells (not shown), to display an image corresponding to the image signal transmitted from the signal processing module 100 into a real image. The PDP module 200 loads and stores the update program transmitted from the signal processing module 100. The PDP module 200 includes a power supply (not shown), upper and lower modules (not shown) including a plurality of cells formed therein, a driver module (not shown) to drive the PDP module 200, and a logic module 210. The logic module 210 includes a third interfacing unit 212, a second storing unit 214 and a second controlling unit 216.

The third interfacing unit 212 is communicably linked with the first interfacing unit 120. The third interfacing unit 212 transmits the update program including the SDA and the SCL provided from the first interfacing unit 120 to the second storing unit 214, and the first control signal CTL and the second control signal CTL to the second controlling unit 216.

The second storing unit 214 stores the application program of the PDP module 200 and stores the update program provided by the external apparatus 100a through the third interfacing unit 212, to update the previously stored program.

The second controlling unit 216 determines a current state based on the control signal transmitted through the third interfacing unit 212. That is, when the first control signal CTL is received, the second controlling unit 216 determines that the PDP module 200 is selected and the update program is transmitted. Accordingly, the second controlling unit 216 updates the application program stored in the second storing unit 214 using the update program. The second controlling unit 216 determines that is should do routine work without updating the application program, when the second control signal CTL is received.

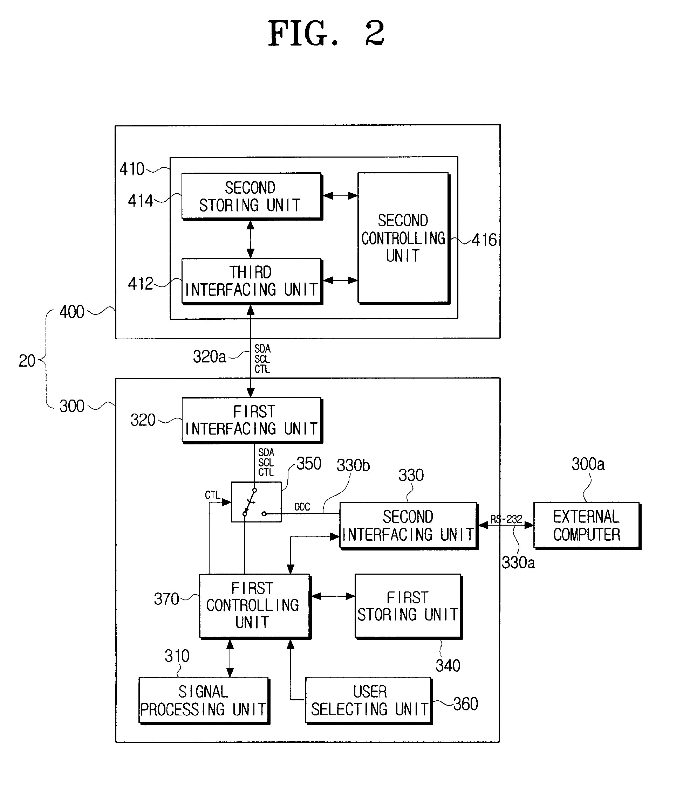

Figure 2 is a block diagram of a PDP (plasma display panel) apparatus according to a second embodiment of the present invention.

Referring to Figure 2, the PDP apparatus 20 capable of updating according to the second embodiment of the present invention, includes a signal processing module 300 and a PDP module 400, and updates a program of the signal processing module 300 or the PDP module 400 using an update program provided from an external computer 300a.

The external computer 300a, employed as an external apparatus, stores the update program of the signal processing module 300 and/or the update program of the PDP module 400. The stored update program is transmitted to the signal processing module 300 through a serial recommended standard-232 (RS-232) cable 330a or a universal serial bus (not shown).

The signal processing module 300 receives the update program of the signal processing module 300 or the PDP module 400 from the external computer 300a according to the mode of operation of the PDP apparatus 20, and transmits the update program of the PDP module 400 to the PDP module 400.

The signal processing module 300 includes a signal processing unit 310, a first interfacing unit 320, a second interfacing unit 330, a first storing unit 340, a switching unit 350, a user selecting unit 360 and the first controlling unit 370. The signal processing unit 310, the first interfacing unit 320, the first storing unit 340, the switching unit 350, the user selecting unit 360 and the first controlling unit 370 operate in a similar manner to the signal processing unit 110, the first interfacing unit 120, the first storing unit 140, the switching unit 150, the user selecting unit 160 and the first controlling unit 170 of Figure 1, such that detailed descriptions thereof will be omitted. The second interfacing unit 330 may be employed with an RS-232 jack or a universal serial bus port. The second interfacing unit 330 and the switching unit 350 may be connected through a general I2C line.

The PDP module 400 loads and stores the update program being transmitted through the signal processing module 300. The PDP module 400 includes a logic module 410 including a third interfacing unit 412, a second storing unit 414 and a second controlling unit 416. Each components 412, 414 and 416 of the logic module 410 are similar to the components 212, 214 and 216 of Figure 1, such that detailed descriptions thereof will be omitted.

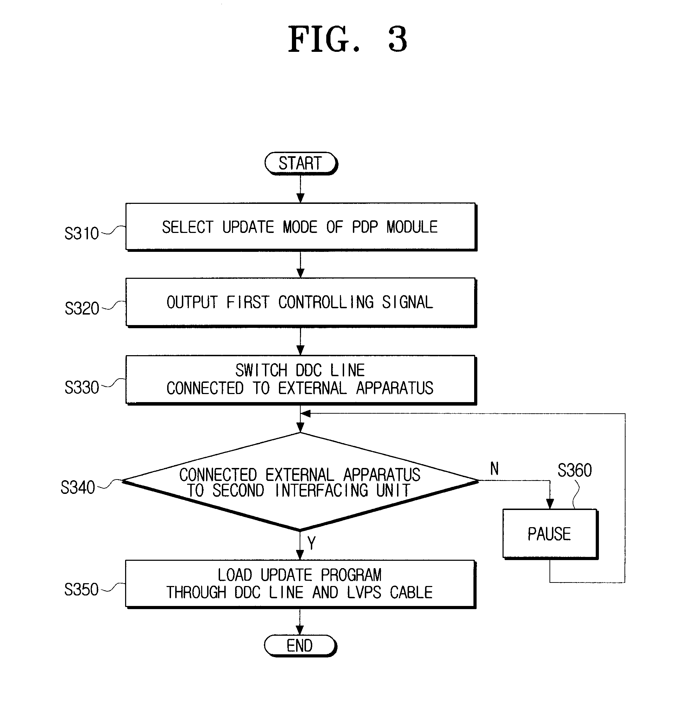

Figure 3 is a flowchart of an updating method of the PDP apparatus capable of being updated according to the first embodiment of the present invention.

Referring to Figures 1 and 3, if the first mode is selected via the user selecting unit 160 in operation S310, to update the PDP module 200, the first controlling unit 170 sets the PDP displaying apparatus 10 to the first mode, and outputs the first control signal CTL to the switching unit 150 and the PDP module 200 in operation S320.

The switching unit 150 then connects the first interfacing unit 120 to the DC line 130b to the second interfacing unit 130 (S330) connected to the external apparatus 100a.

If the second interfacing unit 130 and the external apparatus 100a are connected (S340), the first controlling unit 170 provides the PDP module 200 with the update program of the PDP module 200 received from the external apparatus 100a (S350). More particularly, the first controlling unit 170 controls the update program to be transmitted to the PDP module 200 through the second interfacing unit 130, the DDC line 130b, the switching unit 150, the first interfacing unit 120 and the LVPS cable 120a (S350).

In operation S340, if the second interfacing unit 130 and the external apparatus 100a are not connected, the first controlling unit 170 pauses until the second interfacing unit 130 and the external apparatus 100a are connected (S360). If the second interfacing unit 130 and the external apparatus 100a are not connected, the first controlling unit 170 generates a warning sound and/or a message to inform the user to connect the second interfacing unit 130 and the external apparatus 100a.

According to the first and the second embodiments of the present invention, the signal processing modules 100 and 300 provide a communication path between the external apparatus 100a and the PDP module 200, and a communication path between the external apparatus 300a and the PDP module 400, to update the programs of the PDP modules 200 and 400 without separating the PDP modules 200 and 400 from the signal processing modules 100 and 300.

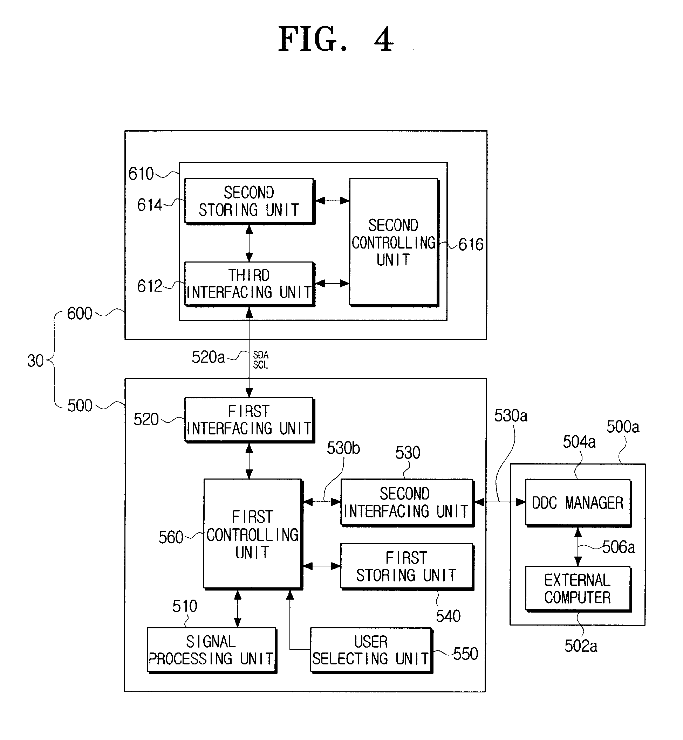

Figure 4 is a block diagram of a PDP apparatus according to a third embodiment of the present invention.

Referring to Figure 4, the PDP apparatus 30 according to the third embodiment of the present invention, includes a signal processing module 500 and a PDP module 600. The PDP apparatus 30 stores an update program provided from an external 500a in the signal processing module 500, and updates the programs of the signal processing module 500 and/or the PDP module 600.

An external apparatus 500a includes an external computer 502a which stores the update program of the signal processing module 500 or the PDP module 600, and a DDC manager 504a performing a relay role. The external computer 502a and the DDC manager 504a are similar to the external computer 102a and the DDC manager 104a of Figure 1, such that detailed descriptions thereof will be omitted.

The signal processing module 500 receives and stores the update program of the signal processing module 500 or the PDP module 600 provided by the external apparatus 500a. When there is a request for updating the PDP module 600, the signal processing module 500 transmits the stored update program of the PDP module 600 to the PDP module 600.

The signal processing module 500 includes a signal processing unit 510, a first interfacing unit 520, a second interfacing unit 530, a first storing unit 540, a user selecting unit 550 and a first controlling unit 560. The signal processing unit 510, the first interfacing unit 520, the second interfacing unit 530 and the user selecting unit 550 are similar to the signal processing unit 110, the first interfacing unit 120, the second interfacing unit 130 and the user selecting unit 160 of the Figure 1, such that detailed descriptions thereof will be omitted.

The first storing unit 540 is divided into at least two areas. The first area stores an application program of the signal processing module 500 and the second area stores the update program of the PDP module 600 provided by the external apparatus 500a. The first storing unit 140 may, for example, be a flash memory or an SDRAM.

If a mode selecting signal for updating the PDP module 600 is received from the user selecting unit 550 and the update program of the PDP module 600 is received from the external apparatus 500a, the first controlling unit 560 controls the first storing unit 540 such that the received update program of the PDP module 600 is stored in the second area of the first storing unit 540. In particular, the update program of the PDP module 600 is received through the computer cable 530a, the second interfacing unit 530, a DDC line 530b and the first controlling unit 560 and stored in the second area of the first storing unit 540.

If a request for updating the PDP module 600 is received from the user selecting unit 550, the first controlling unit 560 extracts the update program stored in the second area and controls the first interfacing unit 520, to transmit the extracted update program to the PDP module 600. The first controlling unit 560 transmits the update program of the PDP module 600 including data (SDA) and a clock signal (SCL), in accordance with I2C standards to the PDP module 600.

The PDP module 600 includes a logic module 610 including a third interfacing unit 612, a second storing unit 614 and a second controlling unit 616. The logic module 610 controls on and off operations of cells arranged on the PDP module 600.

The third interfacing unit 612 receives the update program of the PDP module 600 from the first interfacing unit 612 and provides the update program of the PDP module 600 to the second storing unit 614.

The second controlling unit 616 controls the second storing unit 614 to update the existing program using the update program transmitted through the third interfacing unit 612.

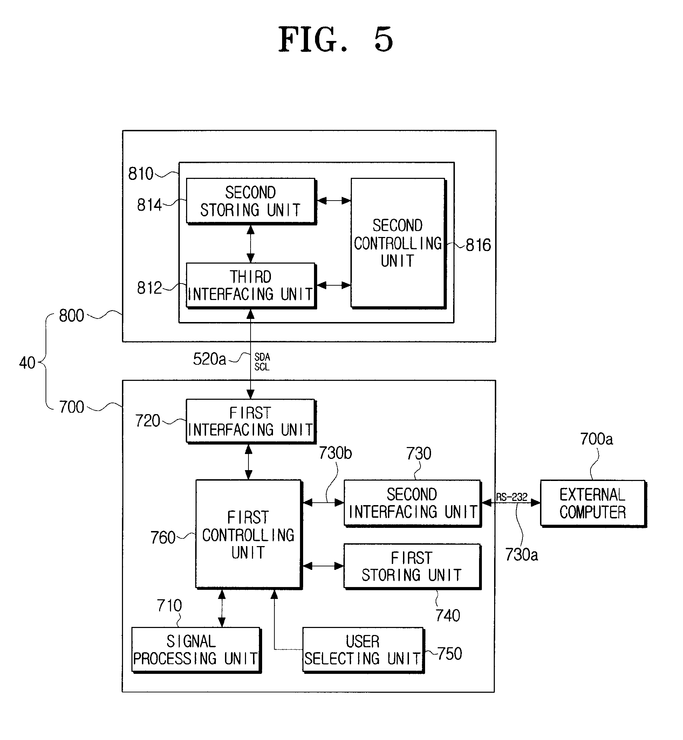

Figure 5 is a block diagram of a PDP apparatus according to a fourth embodiment of the present invention.

Referring to Figure 5, the PDP apparatus 40 according to the fourth embodiment of the present invention, includes a signal processing module 700 and a PDP module 800, and updates a program of the signal processing module 700 or the PDP module 800 using an update program provided from an external computer 700a.

The external computer 700a stores the update program of the signal processing module 700 and/or the update program of the PDP module 800. The stored update programs are transmitted to the signal processing module 700 through a serial RS-232 cable 730a or a universal serial bus (not shown).

The signal processing module 700 receives the update program of the signal processing module 700 or the PDP module 800 from the external computer 700a and stores the update program of the signal processing module 700 or the PDP module 800. If a request for updating the PDP module 800 is received, the signal processing module 700 transmits the stored update program of the PDP module 800 to the PDP module 800.

The signal processing module 700 includes a signal processing unit 710, a first interfacing unit 720, a second interfacing unit 730, a first storing unit 740, a user selecting unit 750, and a first controlling unit 760. The signal processing unit 710, the first interfacing unit 720 and the user selecting unit 750 are similar to the signal processing unit 110, the first interfacing unit 120 and the user selecting unit 160 of Figure 1. The first storing unit 740 and the first controlling unit 760 are similar to the first storing unit 540 and the first controlling unit 560 of Figure 4. Accordingly, detailed descriptions of these components will be omitted.

The second interfacing unit 730 may be an RS-232 jack or a universal serial bus port.

The first storing unit 740 is divided into at least two areas. The first area stores an application program of the signal processing module 700, and the second area stores the update program of the PDP module 800 provided by the external apparatus 700a.

If a mode selecting signal for updating the PDP module 800 is received from the user selecting unit 750 and the update program of the PDP module 800 is received from the external apparatus 700a, the first controlling unit 760 controls the first storing unit 740 to store the received update program in the second area of the first storing unit 740. The update program of the PDP module 600 is received through an RS-232 cable 730a, the second interfacing unit 730 and the first controlling unit 760 and stored in the second area of the first storing unit 740.

If a request for updating the PDP module 800 is received from the user selecting unit 750, the first controlling unit 760 reads the update program stored in the second area and controls the first interfacing unit 720, and transmits the read update program to the PDP module 800. The first controlling unit 760 provides the update program of the PDP module 800 in the form of data (SDA) and a clock signal (SCL).

The PDP module 800 includes a logic module 810 including a third interfacing unit 812, a second storing unit 814 and a second controlling unit 816. The logic module 810 controls on and off operations of cells arranged on the PDP module 800.

The third interfacing unit 812 provides the application program of the PDP module 800 received from the first storing unit 740 to the second storing unit 740.

The second controlling unit 816 controls the second storing unit 814 to update an existing program using the update program received through the third interfacing unit 812.

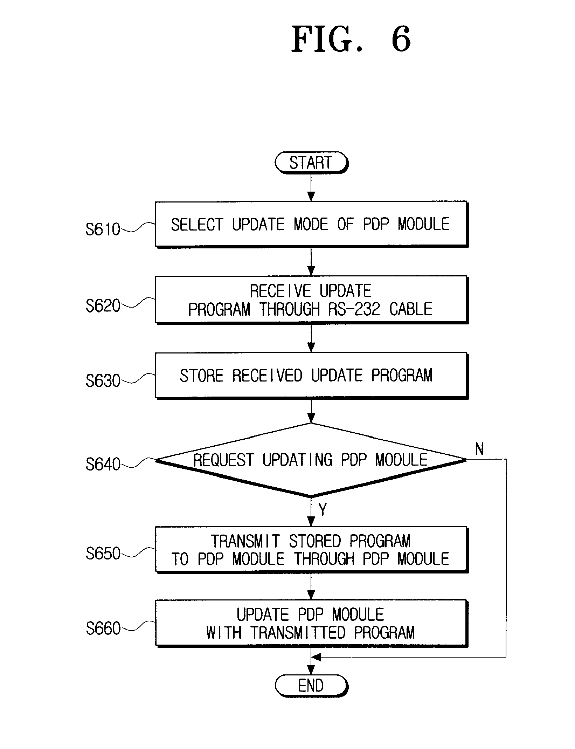

Figure 6 is a flowchart of an updating method of the PDP apparatus according to the third embodiment of the present invention.

Referring to Figures 4 and 6, if the mode selecting signal is received from the user selecting unit 550 for updating the PDP module 600 (S610), the first controlling unit 560 receives the update program of the PDP module 600 through the RS-232 cable 530a and stores the received update program in the second area of the first storing unit 540 (S620 and S630).

If a request for updating the PDP module 600 is received from the user selecting unit 550 (S640), the first controlling unit 560 reads the update program of the PDP module 600 stored in the first storing unit 540, and then, controls the second interfacing unit 530, to transmit the read update program to the PDP module 600 through the LVPS cable 520a (S650). The second controlling unit 616 controls the second storing unit 614 to update the existing program using the update program received by the third interfacing unit 612 (S660).

According to the third and the fourth embodiments of the present invention, the signal processing modules 500 and 700 receive the update programs of the PDP modules 600 and 800 from the external apparatuses 500a and 700a, and provide the update programs to the PDP modules 600 and 800, when there is a request. Accordingly, it is possible to update the programs of the PDP modules 600 and 800, without separating the PDP modules 600 and 800 from the signal processing modules 500 and 700.

The signal processing boards 100, 300, 500 and 700 according to embodiments of the present invention can be formed on a single printed circuit board (PCB).

The embodiments of the present invention may be generally implemented by a developer via a factory mode of operation. The factory mode is used for the developer to update a product or give an after sales service, and the update mode is part of the factory mode.

According to the PDP apparatus and the updating method thereof, it is possible to update the PDP modules 200, 400, 600 and 800 without separating the PDP modules 200, 400, 600 and 800 from the signal processing module 100, 300, 500 and 700, when the PDP modules 200, 400, 600 and 800 are updated in developing or completing the PDP display apparatus. That is, the PDP modules can be updated while connected to the signal processing boards.

More particularly, according to embodiments of the present invention, it is possible to provide the PDP modules 200 and 400 with the update programs of the PDP modules 200, 400 received from the external apparatuses 100a and 300a, by connecting the external apparatuses 100a and 300a to the PDP modules 200 and 400 by use of a switch, when the switching units 150 and 350 are formed on the signal processing modules 100 and 300 and it is requested to update the PDP modules 200, 400 in the factory mode. Accordingly, it is possible to simply update the program by a switching operation, without implementing a complicated circuit, using a separate download tool or separating the PDP modules 200 and 400 from the signal processing modules 100 and 300.

According to the embodiments of the present invention, the signal processing modules 100, 300, 500 and 700 may include a storing medium such as an SDRAM, and store the update programs of the PDP modules 200, 400, 600 and 800 provided from the external apparatus 100a, 300a, 500a and 700a. The PDP apparatus may update the programs of the PDP modules 200, 400, 600 and 800, by providing the PDP modules 200, 400, 600 and 800 with the update programs stored at a later time without separating the PDP modules 200, 400, 600 and 800 from the signal processing module even after manufacturing the PDP apparatus.

It is possible to inexpensively and easily implement the present invention, by adopting widely used interfaces such as a service port, a universal serial bus port, a computer jack or an RS-232 jack without requiring a separate port or terminal on the PDP apparatus.

While the invention has been shown and described with reference to certain embodiments thereof, it will be understood by those skilled in the art that various changes in form and details may be made therein without departing from the scope of the invention as defined by the appended claims.

## Claims

1. A plasma display panel (PDP) apparatus comprising:
a PDP module for displaying an image; and
a signal processing module for providing an image signal to the PDP module, arranged to receive an update program for the PDP module from an external apparatus, and to provide the program to the PDP module.

2. The PDP apparatus of claim 1, wherein the signal processing module comprises:
means for receiving an update request;
a first interfacing unit arranged to communicate with the PDP module;
a second interfacing unit arranged to communicate with the external apparatus;
a first controlling unit arranged to output a first control signal if a PDP module update request is received by the PDP apparatus; and
a switching unit arranged to connect the first interfacing unit to the second interfacing unit to provide a communication path between the external apparatus and the PDP module if the first control signal is received from the first controlling unit.

3. The PDP apparatus of claim 2, wherein the first controlling unit is arranged to output a second control signal if a signal processing module update request is received by the PDP apparatus, and the switching unit is arranged to disconnect the communication path between the external apparatus and the PDP module and to receive an update program provided from the external apparatus through the second interfacing unit.

4. The PDP apparatus according to claim 1 in which the signal processing module is arranged to store the update program for the PDP module on receiving it from the external apparatus.

5. The PDP apparatus of claim 4, wherein the signal processing module comprises:
means for receiving an update request;
a first interfacing unit arranged to communicate with the PDP module;
a second interfacing unit arranged to communicate with the external apparatus; and
a controlling unit arranged to receive the update program for the PDP module from the external apparatus via the second interfacing unit and to store the program in the storing unit, if a PDP module update request is received by the PDP apparatus.

6. The PDP apparatus of claim 5, wherein the controlling unit is arranged to read the update program stored in the storing unit and to provide the update program to the PDP module via the first interfacing unit, if a PDP module update request is received by the PDP apparatus.

7. The PDP apparatus of any one of claims 2 to 6, wherein the PDP module comprises:
a third interfacing unit arranged to receive the update program provided from the external apparatus through the signal processing module;
a storing unit arranged to store the received update program; and
a second controlling unit arranged to control the third interfacing unit and the storing unit to receive and store the update program.

8. The PDP apparatus of any preceding claim, wherein the signal processing module is connected to the external apparatus through a computer cable, and the external apparatus comprises:
a computer for providing the update program; and
a display data channel manager arranged to receive the update program from the computer and to transmit it to the signal processing module via the computer cable.

9. The PDP apparatus of any one of claims 1 to 7, wherein the signal processing module is connected to the external apparatus through one of a recommended standard (RS)-232 cable and a universal serial bus, and the external apparatus comprises a computer for storing the update program.

10. A method of updating a PDP apparatus which comprises a PDP module for displaying an image, and a signal processing module arranged to communicate with the PDP module, the method comprising:
receiving an update program for the PDP module from an external apparatus at the signal processing module and providing the program to the PDP module;
storing the update program in the PDP module; and
updating the PDP module using the update program.

11. The method of claim 10, the PDP module is connected to the external apparatus through a switching unit of the signal processing module, if a PDP module update request is received by the PDP apparatus.

12. The method of claim 10 in which the receiving of the update program further comprises storing the update program in the signal processing module, and the program is transmitted to the PDP module and the PDP module is updated if a PDP module update request is received.

13. The method of any one of claims 10 to 12, wherein the signal processing module is connected to the external apparatus through one of a computer cable, a recommended standard (RS)-232 cable and a universal serial bus, and the signal processing module is connected to the PDP module through a cable which complies with an Inter-IC communication standard.
